# EUROPEAN PATENT APPLICATION

(11) **EP 4 751 682 A1**
(43) Date of publication of application: **03.06.2026**
(21) Application number: 24844759.1
(22) Date of filing: 22.07.2024
(51) Int. Cl.: A61F 2/06, A61F 2/24

(54) **SHUNT DEVICE**

(30) Priority: 24.07.2023 CN 202310908470
(71) Applicant: United Innomed (Shanghai) Limited, Shanghai 201315 (CN)
(72) Inventor: GE, Shuchen, Shanghai 201315 (CN); WANG, Li, Shanghai 201315 (CN); LI, Bo, Shanghai 201315 (CN)
(74) Representative: Murgitroyd & Company
(86) International application number: PCT/CN2024/106750
(87) International publication number: WO 2025/021068

(57) **Abstract**

The present application provides a shunt device, including a shunt component and a flow regulation mechanism. The shunt component includes a body, and an inlet and an outlet located at both ends of the body, and the shunt component defines a shunt channel for a pressurized fluid. The flow regulation mechanism is connected to the shunt component and is configured to regulate a flow rate of the pressurized fluid flowing through the shunt channel. The flow regulation mechanism includes a deformable blocking portion configured to, when a fluid pressure at the inlet of the shunt component and/or inside the shunt component is less than a fluid pressure external to the flow regulation mechanism, be driven by the fluid pressure external to the flow regulation mechanism to collapse radially, thereby causing the flow regulation mechanism to partially close the shunt channel.

## Description

### TECHNICAL FIELD

The present application relates to the field of interventional medical devices, and in particular, to a shunt device.

### BACKGROUND

Heart failure (HF) is a clinical syndrome caused by abnormalities in ventricular filling and ejection functions. The main manifestations of heart failure include dyspnea, fatigue, fluid retention (pulmonary congestion, systemic venous congestion, and peripheral edema), and so on. Heart failure can be classified into three categories: heart failure with reduced ejection fraction, heart failure with mildly reduced ejection fraction, and heart failure with preserved ejection fraction. In patients with chronic heart failure with preserved or reduced ejection fraction, persistently elevated left atrial pressure is a primary cause of pulmonary congestion, accounting for 90% of hospital admissions among HF patients.

Atrial septal shunting is a means to reduce elevated left atrial pressure. It involves creating an aperture in the interatrial septum to guide blood flow from the left atrium to shunt into the right atrium, thereby reducing the load on the left atrium. An atrial shunt device can establish a passage between the left atrium and the right atrium by defining the diameter of the interatrial aperture, effectively lowering the patient's left atrial pressure and alleviating pulmonary congestion and dyspnea. After implantation of the atrial shunt device, when the left atrial pressure is higher than the right atrial pressure, blood does not flow back from the right atrium to the left atrium. However, when the patient's right atrial pressure exceeds that of the left atrium during certain periods (e.g., breath-holding, Valsalva maneuver), or when the pressures in the two atria are similar, undesirable backflow may occur. This results in a right-to-left atrial shunt, allowing fine "particles" (e.g., small thrombi) from the venous circulation to enter the left atrium. This increases the risk of migraines or paradoxical embolism, augments left atrial filling, and reduces blood oxygen content.

Therefore, in some existing atrial shunt device designs, a one-way valve structure is incorporated within the shunt. It opens only when the left atrial pressure is higher than the right atrial pressure, allowing blood to flow from the left atrium to the right atrium, and closes completely when the left atrial pressure is lower than the right atrial pressure, preventing backflow from the right to atrium the left atrium. However, the inventors have found that this design introduces a new problem in practical use: the one-way valve structure is prone to failure after a period of time in vivo, becoming unable to open in response to the pressure difference, thereby causing the shunt device to fail.

### SUMMARY

In view of the foregoing, the present application provides a shunt device to address one or more of the problems in the prior art.

In a first aspect, a shunt device is provided. The shunt device is used for shunting a pressurized fluid, and the shunt device includes a shunt component and a flow regulation mechanism. The shunt component includes a body, and an inlet and an outlet located at both ends of the body, and the shunt component defines a shunt channel for the pressurized fluid. The flow regulation mechanism is connected to the shunt component and is configured to regulate a flow rate of the pressurized fluid flowing through the shunt channel. The flow regulation mechanism includes a deformable blocking portion configured to be driven by a fluid pressure external to the flow regulation mechanism to collapse radially when a fluid pressure at the inlet of the shunt component and/or inside the shunt component is less than the fluid pressure external to the flow regulation mechanism.

In a possible implementation, the flow regulation mechanism is allowed to never completely close the shunt channel.

In a possible implementation, the blocking portion is configured to vary a flow cross-sectional area of the shunt channel. The flow regulation mechanism has a first state and a second state. In the first state, the blocking portion is in a collapsed configuration such that the shunt channel has a first flow cross-sectional area. In the second state, the blocking portion is in an open configuration such that the shunt channel has a second flow cross-sectional area. The first flow cross-sectional area is greater than zero and is less than the second flow cross-sectional area. The blocking portion is configured to, when a fluid pressure at the inlet of the shunt component and/or inside the shunt component is less than the fluid pressure external to the flow regulation mechanism, be driven by the fluid pressure external to the flow regulation mechanism to collapse radially, thereby causing the flow regulation mechanism to be in the first state.

In a possible implementation, when the blocking portion is in a collapsed configuration, the blocking portion is deformed into a recessed state. When the blocking portion is in an open configuration, the blocking portion is deformed into a bowed state. The blocking portion is configured to, when the fluid pressure at the inlet of the shunt component and/or inside the shunt component is less than the fluid pressure external to the flow regulation mechanism, be driven by the fluid pressure external to the flow regulation mechanism to be recessed radially, thereby causing the flow regulation mechanism to be in the first state.

With reference to the foregoing possible implementations, in another possible implementation, the flow regulation mechanism includes an adjustment flexible tube connected to the shunt component. A tube wall of the adjustment flexible tube includes a first portion distributed in a circumferential direction. The first portion is configured to be recessed radially or bowed radially. The first portion is the blocking portion.

With reference to the foregoing possible implementations, in another possible implementation, the adjustment flexible tube further includes a second portion. The second portion and the first portion are distributed spaced apart from each other in the circumferential direction. The flow regulation mechanism further includes a shaping frame. The shaping frame is configured to maintain the second portion in a bowed state and to allow the first portion to be controllably recessed or bowed with a constraint of the shaping frame by supporting a predetermined portion of the adjustment flexible tube.

With reference to the foregoing possible implementations, in another possible implementation, the shaping frame includes a plurality of fixing portions, an end of the fixing portion is connected to the shunt component, another end of the fixing portion extends in an axial direction, and the plurality of fixing portions are distributed in a circumferential direction of the adjustment flexible tube; and a tube wall of the adjustment flexible tube includes a plurality of first portions and a plurality of second portions corresponding to the plurality of fixing portions, the first portion is disposed between two circumferentially adjacent fixing portions, and the second portion is fixed to the fixing portion and has a predetermined width, such that parts of two adjacent first portions that are proximate to the fixing portion remain out of contact with each other.

With reference to the foregoing possible implementations, in another possible implementation, a width of the fixing portion corresponds to a width of the second portion. The adjustment flexible tube is disposed at an inner side and/or an outer side of the fixing portion.

With reference to the foregoing possible implementations, in another possible implementation, the fixing portion includes a first vertical rod and a second vertical rod. A predetermined spacing is provided between the first vertical rod and the second vertical rod. An end of the first vertical rod and an end of the second vertical rod are connected to the shunt component. Another end of the first vertical rod and another end of the second vertical rod gradually converge along extension directions of the first vertical rod and the second vertical rod.

With reference to the foregoing possible implementations, in another possible implementation, the fixing portion further includes a horizontal rod connecting the another end of the first vertical rod and the another end of the second vertical rod, a reinforcement rib disposed between the first vertical rod and the second vertical rod, and an annular frame connecting the end of the first vertical rod and the end of the second vertical rod.

With reference to the foregoing possible implementations, in another possible implementation, the plurality of fixing portions includes three fixing portions.

With reference to the foregoing possible implementations, in another possible implementation, the three fixing portions are uniformly distributed in the circumferential direction of the adjustment flexible tube.

With reference to the foregoing possible implementations, in another possible implementation, a hanging lug is provided at distal end of the fixing portion, and the hanging lug is configured to connect to a predetermined structure within an interventional catheter.

With reference to the foregoing possible implementations, in another possible implementation, an edge contour of the first portion is a curve recessed toward the inlet, an edge contour of the second portion is a curve smoothly connected to the edge contour of the first portion, and a part of the first portion that is distal from the fixing portion has an axial dimension that is less than an axial dimension of a part of the first portion that is proximate to the fixing portion.

With reference to the foregoing possible implementations, in another possible implementation, an edge contour of the first portion is a curve and has a minimum length dimension at an intermediate position, an edge contour of the second portion is a curve connected to the edge contour of the first portion and has a maximum length dimension at an intermediate position, and a ratio of the minimum length dimension to the maximum length dimension ranges from 0.3 to 1.

With reference to the foregoing possible implementations, a ratio of the minimum length dimension to a diameter of the adjustment flexible tube ranges from 0.5 to 3.

With reference to the foregoing possible implementations, in another possible implementation, there are a plurality of first portions, the plurality of first portions, when recessed, maintain incomplete contact at a center of the shunt channel, thereby dividing the shunt channel into a plurality of sub-channels, and the plurality of sub-channels are located at an edge of the shunt channel.

With reference to the foregoing possible implementations, in another possible implementation, there are a plurality of first portions, and the plurality of first portions, when recessed, maintain out of contact with each other and form a slit.

With reference to the foregoing possible implementations, in another possible implementation, the shunt component and the shaping frame are formed as a metal framework by unitary winding of a wire, and any cross-section of the metal frame has a non-enclosed contour.

With reference to the foregoing possible implementations, in another possible implementation, the shaping frame is integrally formed with the blocking portion; or the shaping frame is connected to the blocking portion as a separate component, and the separate component is formed by cutting a metal tube or by bending a metal wire.

With reference to the foregoing possible implementations, in another possible implementation, the body includes a holder and a shunt flexible tube disposed at an inner surface and/or an outer surface of the holder, the shunt flexible tube and the adjustment flexible tube are integrally formed, the shunt component is connected to the flow regulation mechanism at the outlet, the body and the shunt flexible tube form a first segment of the shunt channel, and the adjustment flexible tube forms a second segment of the shunt channel.

With reference to the foregoing possible implementations, in another possible implementation, the holder includes a clamping device, the clamping device includes a plurality of pairs of a first clamping arm and a second clamping arm, and the first clamping arm and the second clamping arm respectively extend from a shunt portion of the holder.

With reference to the foregoing possible implementations, in another possible implementation, the first clamping arm and the second clamping arm respectively extend from the shunt portion of the holder and gradually converge toward each other.

With reference to the foregoing possible implementations, in another possible implementation, first clamping arms and/or second clamping arms each includes two extension wire segments and an annular wire segment, and two ends of the annular wire segment are respectively connected to one extension wire segment.

With reference to the foregoing possible implementations, in another possible implementation, the flow regulation mechanism further includes a shaping frame, the shaping frame includes a plurality of elastic guide frames, and the elastic guide frames are connected to the blocking portion and are configured to maintain the blocking portion in the collapsed configuration in an initial state.

With reference to the foregoing possible implementations, in another possible implementation, an end of the elastic guide frame is connected to the body, another end of the elastic guide frame forms an elastic cantilever in an axial direction, and the another end is closer to a center of the shunt channel than the end.

With reference to the foregoing possible implementations, in another possible implementation, the flow regulation mechanism includes an adjustment flexible tube connected to the shunt component, a tube wall of the adjustment flexible tube includes a first portion and a second portion distributed in a circumferential direction, and the second portion and the first portion are distributed spaced apart from each other in the circumferential direction; and the first portion is configured to be capable of being recessed radially or bowed radially, the first portion is the blocking portion, and the elastic guide frames are configured to maintain the second portion in a constantly bowed state.

With reference to the foregoing possible implementations, in another possible implementation, the elastic guide frame is formed by bending a metal wire and, when subjected to a radially outward force, is capable of being parallel to a central axis of the shunt component.

With reference to the foregoing possible implementations, in another possible implementation, the shaping frame is integrally formed with the blocking portion; or the shaping frame is connected to the blocking portion as a separate component, and the separate component is formed by cutting a metal tube or by bending a metal wire.

With reference to the foregoing possible implementations, in another possible implementation, there are a plurality of blocking portions, and an initial state of the flow regulation mechanism is the first state or the second state.

With reference to the foregoing possible implementations, in another possible implementation, the shunt component further includes a shunt flexible tube disposed at an inner surface and/or an outer surface of the body. The shunt flexible tube and the adjustment flexible tube are integrally formed. The shunt component is connected to the flow regulation mechanism at the outlet. The body and the shunt flexible tube form a first segment of the shunt channel. The adjustment flexible tube forms a second segment of the shunt channel.

With reference to the foregoing possible implementations, in another possible implementation, the shunt device further includes a monitoring device, and the monitoring device is connected side by side with the shunt component.

In the shunt device provided in the present application, the flow regulation mechanism is disposed on the shunt channel defined by the shunt component. The flow regulation mechanism can switch between the first state and the second state under an action of the pressurized fluid flowing through the shunt channel, thereby restricting the flow rate of the pressurized fluid in an undesired flow direction while permitting the pressurized fluid to pass in a desired flow direction. In the present application, the first state corresponds to a state where the flow regulation mechanism is in a flow-restricting state, and the second state corresponds to a state where the flow regulation mechanism is in a flow-permitting state. Unlike a complete closure design of a check valve structure in the prior art for preventing backflow, the flow regulation mechanism of the present application never completely closes the flow channel. While ensuring that minor backflow does not affect normal physiological functions of human body, a partial closure design effectively avoids a problem of failure due to neointimal hyperplasia, ensures an intended shunting function of the shunt device, reduces probability of heart failure in patients due to failure of the shunt device, and effectively improves patient survival rates.

### BRIEF DESCRIPTION OF THE DRAWINGS

To illustrate technical solutions of embodiments of the present application more clearly, the drawings used in the embodiments will be briefly described below.

It should be understood that the following drawings illustrate only some embodiments of the present application and should not be considered as limiting the scope.

It should also be understood that the same or similar reference numerals are used in the drawings to denote the same or similar components.

It should also be understood that the drawings are schematic only, and the dimensions and proportions of the components in the drawings are not necessarily precise.
FIG. 1 is a schematic structural diagram of a shunt device according to an embodiment of the present application.
FIG. 2 is a schematic structural diagram of an end face of the flow regulation mechanism of FIG. 1 at an outlet side.
FIG. 3 is a schematic diagram of a flow regulation mechanism in a second state according to an optional implementation.
FIG. 4 is a schematic diagram of the flow regulation mechanism of FIG. 3 in a first state.
FIG. 5 is a schematic structural diagram of a shunt device according to another embodiment of the present application, where the flow regulation mechanism is in a first state.
FIG. 6a is a schematic structural diagram of a shunt device according to another embodiment of the present application, where the flow regulation mechanism is in a first state.
FIG. 6b is a cross-sectional view taken along the line B-B in FIG. 6a.
FIG. 7 is a schematic structural diagram of the flow regulation mechanism of the shunt device shown in FIG. 6a in a second state.
FIG. 8a is a schematic structural diagram of a flow regulation mechanism according to an optional implementation.
FIG. 8b is a schematic structural diagram of a flow regulation mechanism according to another optional implementation.
FIG. 8c is a schematic structural diagram of a flow regulation mechanism according to another optional implementation.
FIG. 8d is a schematic structural diagram of a flow regulation mechanism according to another optional implementation.
FIG. 9 is a schematic structural diagram of a shunt device according to another embodiment of the present application.
FIG. 10 is a schematic structural diagram of a shunt device according to another embodiment of the present application.
FIG. 11 is a three-dimensional schematic structural diagram of the shunt device shown in FIG. 10.
FIG. 12 is a schematic structural diagram of the second portion of the adjustment flexible tube in the shunt device shown in FIG. 11 when the flow regulation mechanism is in a first state.
FIG. 13 is a schematic structural diagram of a shunt device according to another embodiment of the present application.

List of reference numerals:
10-shunt, and 20-sensor;
100-shunt component, 110-holder, 111-first clamping arm, 112-second clamping arm, 113-inlet, 114-outlet, and 120-shunt flexible tube; and
200, 200a-flow regulation mechanism, 210, 210a, 210f-shaping frame, 211, 211a-fixing portion, 213, 213a, 213f-first vertical rod, 214, 214a, 214f-second vertical rod, 215-horizontal rod, 216-hanging lug, 217-reinforcement rib, 220, 220a, 220f-adjustment flexible tube, 221, 221a, 221b, 221c, 221d, 221e, 221f-first portion, 222, 222a, 222b, 222c, 222d, 222e, 222f-second portion, 230-sub-channel, and 211b, 211c, 211d, 211e, 211f-elastic guide frame.

### DETAILED DESCRIPTIONS OF THE EMBODIMENTS

Embodiments of the present application are exemplarily described below with reference to the accompanying drawings. It should be understood that the present application can be implemented in many forms and should not be construed as limited to the embodiments set forth herein, which are provided for a more thorough and complete understanding of the present application.

It should be understood that the terms "including" and variations thereof as used in the present application are open-ended, meaning "including but not limited to". The term " based on" means "at least partially based on". The term "a plurality of" means "two or more".

It should be understood that although the terms such as "first" or "second" are used in the present application to describe various elements, and these elements are not limited by these terms. These terms are used only to distinguish one element from another.

Embodiments of the present application provide a shunt device for shunting a pressurized fluid, which is, for example, disposed at an interatrial septum of a heart to guide part of the blood from the left atrium to the right atrium. The pressurized fluid herein refers to a fluid driven by a pressure difference, which, in the embodiments, is the blood. The shunt device includes a shunt component and a flow regulation mechanism. The shunt component includes a body, and an inlet and an outlet located at both ends of the body. The flow regulation mechanism is connected to the shunt component. The shunt component defines a shunt channel for the pressurized fluid. The flow regulation mechanism is configured to regulate a flow rate of the pressurized fluid flowing through the shunt channel. When a pressure in a left atrium is greater than a pressure in a right atrium, a flow cross-sectional area of the shunt channel increases in response to an increase in a fluid pressure until reaching a maximum flow cross-sectional area. When the pressure in the right atrium is greater than a pressure in a left atrium, a flow cross-sectional area of the shunt channel decreases, thereby restricting a flow rate of a fluid from the outlet toward the inlet.

Further, the flow regulation mechanism includes a deformable blocking portion. The blocking portion is configured to vary a flow cross-sectional area of the shunt channel by changing its shape. The flow cross-sectional area herein refers to a cross-sectional area at a narrowest position within the shunt channel, such as an area of an opening formed by an edge contour of a distal end of the flow regulation mechanism. The flow regulation mechanism has a first state and a second state. In the first state, the blocking portion is in a collapsed configuration such that the shunt channel has a first flow cross-sectional area. In the second state, the blocking portion is in an open configuration such that the shunt channel has a second flow cross-sectional area. The first flow cross-sectional area is greater than zero and is less than the second flow cross-sectional area. The blocking portion is configured to, when a fluid pressure at the inlet of the shunt component and/or inside the shunt component is less than a fluid pressure external to the flow regulation mechanism, be driven by the fluid pressure external to the flow regulation mechanism to collapse radially, thereby causing the flow regulation mechanism to be in the first state. Conversely, the blocking portion is configured to, when the fluid pressure at the inlet of the shunt component and/or inside the shunt component is greater than the fluid pressure external to the flow regulation mechanism, be driven by the fluid pressure at the inlet of the shunt component and/or inside the shunt component to open radially, thereby causing the flow regulation mechanism to be in the second state. In the first state, the blocking portion is in the collapsed configuration to block a part of the shunt channel, so that only a small amount of fluid can flow from the outlet toward the inlet. Since the blocking portion does not close completely, a problem of the blocking portion being adhered due to tissue hyperplasia and thus unable to open to the second state is effectively avoided. In different embodiments, an initial state of the flow regulation mechanism may be either the first state or the second state.

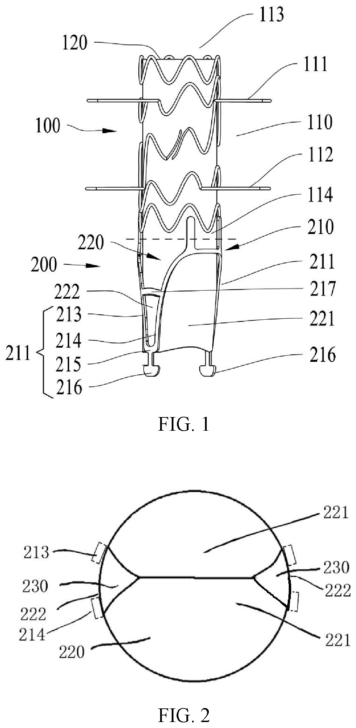

With reference to FIG. 1 and FIG. 2, FIG. 1 is a schematic structural diagram of a shunt device according to an embodiment of the present application, and FIG. 2 is a schematic structural diagram of an end face of the flow regulation mechanism of FIG. 1 at an outlet side.

The shunt device includes a shunt component 100 and a flow regulation mechanism 200. The shunt component 100 includes a body formed by a holder 110 and a shunt flexible tube 120. The holder 110 is a cylindrical mesh frame formed by winding/ weaving an elastic metal wire, or by cutting a metal tube, and is provided on its outer periphery with a plurality of pairs of clamping devices, the clamping device includes a first clamping arm 111 and a second clamping arm 112, and the clamping device is used for clamping an interatrial septum. The shunt flexible tube 120 is formed by a covering film laid on an inner surface and/or an outer surface of the holder 110. Preferably, the covering film is made of a polymer material, for example, a polytetrafluoroethylene membrane. Two ends of the shunt component 100 are an inlet 113 and an outlet 114, respectively (in this embodiment, the outlet 114 of the shunt component 100 is directly connected to an inlet of the flow regulation mechanism 200 in a seamless manner, and therefore, its position in the figure is shown schematically). The flow regulation mechanism 200 also has an inlet and an outlet. The flow regulation mechanism 200 is connected to the shunt component 100 at the outlet 114, and the inlet of the flow regulation mechanism 200 is connected to the outlet 114. When the shunt component 100 is implanted at the interatrial septum of the heart, the inlet 113 is located in the left atrium, and the outlet 114 and the flow regulation mechanism 200 are located in the right atrium. When a left atrial pressure is greater than a right atrial pressure, a portion of a blood flow can pass through the shunt component 100 to reach the right atrium. The flow regulation mechanism 200 can hinder blood from the right atrium from entering the left atrium when the right atrial pressure is greater than the left atrial pressure.

In this embodiment, the shunt component 100 defines a shunt channel for the pressurized fluid. The holder 110 and the shunt flexible tube 120 together form a first segment of the shunt channel. The flow regulation mechanism 200 includes a shaping frame 210 and an adjustment flexible tube 220. The shaping frame 210, in combination with the adjustment flexible tube 220, forms a second segment of the shunt channel. The second segment is a channel with a variable flow cross-sectional area, which is connected in series at the outlet 114 of the shunt component 100 and is connected to the first segment, thereby enabling the flow regulation mechanism 200 to restrict the fluid flowing through the outlet 114.

The shaping frame 210 includes one annular frame and two fixing portions 211. An end of the annular frame is used for connecting to the shunt component 100, and another end of the annular frame is connected to the two radially symmetrical fixing portions 211. The fixing portion 211 extends parallel to an axis of the annular frame. The fixing portion 211 is generally n-shaped, and the fixing portion 211 includes a first vertical rod 213, a second vertical rod 214, and a horizontal rod 215. An end of the first vertical rod 213 and an end of the second vertical rod 214 are connected to the annular frame, while another end of the first vertical rod 213 and another end of the second vertical rod 214 are connected to the horizontal rod 215. The first vertical rod 213 and the second vertical rod 214 may also be connected by a reinforcement rib 217. The reinforcement rib 217 can enhance structural stability of the shaping frame 210, thereby preventing the shaping frame 210 from being damaged and unable to fully recover its shape when crimped into a delivery catheter. The two fixing portions 211 face each other, and a spacing between the two fixing portions 211 is equal to a diameter D of the annular frame and the adjustment flexible tube 220.

Preferably, as shown in FIG. 1, a top end of the two fixing portions 211 is further provided with a hanging lug 216. The hanging lug 216 is used to connect the shunt device to a corresponding structure within an interventional catheter, thereby enabling controlled release of the shunt device to a target site. The shaping frame 210 may be formed by cutting a metal tube made of an elastic material. Therefore, when viewed from the end face of the flow regulation mechanism 200, the shaping frame 210 is generally annular in its entirety. A front projection of an end face of the annular frame may be a complete annular structure or a portion of a complete annular structure. As shown in FIG. 1, the annular frame includes two parts of the complete annular structure, where two ends of a part are respectively connected to the first vertical rod 213, and two ends of another part are respectively connected to the second vertical rod 214.

Preferably, the adjustment flexible tube 220 is a tubular structure formed from a covering film. In this embodiment, the adjustment flexible tube 220 and the shunt flexible tube 120 are a tubular structure formed from a same piece of covering film. In some alternative embodiments, the shunt flexible tube 120 and the adjustment flexible tube 220 may be two separate flexible tubes connected together, for example, stitched or bonded together at a position indicated by a dashed line in FIG. 1. The adjustment flexible tube 220 is disposed on the inner surface and/or the outer surface of the shaping frame 210, and a portion of the adjustment flexible tube 220 in contact with the shaping frame 210 is connected to the shaping frame 210.

The adjustment flexible tube 220 includes a first portion 221 and a second portion 222. The first portion 221 is located between the two fixing portions 211, and the second portion 222 is located between the first vertical rod 213 and the second vertical rod 214. The second portion 222 is fixed to the fixing portion 211 and has a predetermined width W, thereby allowing a junction between the second portion 222 and the first vertical rod 213, and a junction between the second portion 222 and the second vertical rod 214 to remain out of intersect with each other, and also allowing parts of the two first portions 221 that are proximate to the fixing portion 211 to remain out of contact with each other. The above arrangement allows the flow regulation mechanism 200 to have the first state and the second state and to switch between them. The first state refers to a restricted flow state of the flow regulation mechanism 200, and the second state refers to a permitted flow state of the flow regulation mechanism 200. Since the first portion 221 is formed by a thin covering film, even a slight change in a blood pressure difference between the left atrium and the right atrium can also cause the first portion 221 to deform radially in response to the pressure change. This results in high sensitivity and strong self-adaptability in flow regulation, while also promptly mitigating a degree of blood backflow from the right atrium to the left atrium.

Specifically, the spacing between the first vertical rod 213 and the second vertical rod 214 of the fixing portion 211 is relatively small and is approximately equal to a width of the second portion fixed thereto. Therefore, when subjected to a radially inward force toward a central axis, the second portion 222 cannot significantly be recessed toward the center. In contrast, a width of the first portion 221 is significantly greater than a straight-line distance between fixing portions 211 on two sides of the first portion 221 (when the adjustment flexible tube 220 is fully deployed in a cylindrical form, the first portion 221 forms a part of a circumferential surface of the cylinder, while the straight-line distance between the fixing portions on two sides of the first portion 221 equals a distance between two side edges of that circumferential surface). Consequently, the first portion 221 is not constrained by the fixing portions 211 and may be recessed toward the central axis when subjected to a radially inward force. The first portion 221 forms the blocking portion. When the first portion 221 is in the collapsed configuration, the first portion 221 is recessed toward the central axis, thereby reducing the flow cross-sectional area of the adjustment flexible tube 220. The adjustment flexible tube 220 is divided by the two fixing portions 211 into two first portions 221 that face each other. When the two first portions 221 are recessed toward the central axis, parts of the two first portions 221 located near the central axis contact each other, thereby blocking the channel at that location. However, parts of the first portions 221 located near the fixing portions 211 are restrained by the first vertical rod 213 and the second vertical rod 214, thereby allowing them to remain out of contact with each other, thus forming two sub-channels 230 with a generally triangular cross-section. The sub-channels 230 allow the two first portions 221 to maintain incomplete contact, thereby preventing them from adhering to each other due to cardiac tissue hyperplasia. Furthermore, a cross-sectional area of the sub-channels 230 is relatively small, so the fluid flow back to the inlet 113 through them does not have a significant impact. In this state, the shunt channel includes the sub-channels 230, and a sum of the cross-sectional areas of the sub-channels 230 constitutes the first flow cross-sectional area of the shunt channel. When the pressurized fluid flows in from the inlet 113 under a pressure difference and forces the first portion 221 to transition to the open configuration, the first portion 221 is bowed away from the central axis, thereby enabling the shunt channel to have an increased second flow cross-sectional area.

The above-described configuration enables the blocking portion to perform the following functions. When the fluid pressure inside the shunt component 100 is less than the fluid pressure external to the flow regulation mechanism 200, the blocking portion is driven by the fluid pressure external to the flow regulation mechanism 200 to be recessed radially, thereby enabling the flow regulation mechanism 200 to be in the restricted flow state, i.e., the first state. When the fluid pressure inside the shunt component 100 is greater than the fluid pressure external to the flow regulation mechanism 200, the blocking portion is driven by the fluid pressure inside the shunt component 100 to open radially, thereby causing the flow regulation mechanism 200 to be in the permitted flow state, i.e., the second state. Herein, the exterior of the flow regulation mechanism 200 is defined relative to the interior communicated to the inlet of the shunt component 100. It is to be understood that in the first state, the fluid pressure acts directly on the outer surface of the blocking portion. By providing the shaping frame 210, deformation of the first portion 221 can be controlled, thereby allowing the first portion 221 to be recessed and bowed in an intended direction, thus avoiding instability caused by random deformation of the adjustment flexible tube. In the initial state, i.e., prior to being implanted in the human body, the blocking portion may assume either the collapsed configuration or the open configuration due to its certain degree of freedom. Accordingly, the flow regulation mechanism 200 may be in either the first state or the second state initially.

In some alternative embodiments, the shunt channel may be formed solely by the holder 110 and the shunt flexible tube 120. The flow regulation mechanism 200 serves only as a flow-control valve at the outlet of the shunt component 100. Alternatively, the flow regulation mechanism 200 is disposed inside the shunt component 100, and the shunt component 100 may also have other structures such as a V-shape or H-shape. When the flow regulation mechanism 200 is disposed inside the shunt component 100, the blocking portion is configured to, when the fluid pressure at the inlet of the shunt component (which can also be understood as the fluid pressure inside the shunt component near the inlet) is less than the fluid pressure external to the flow regulation mechanism (which can also be understood as the fluid pressure inside the shunt component near the outlet), be driven by the fluid pressure external to the flow regulation mechanism to radially collapse, thereby causing the flow regulation mechanism to be in the first state. When the fluid pressure at the inlet of the shunt component is greater than the fluid pressure external to the flow regulation mechanism, the blocking portion is configured to be driven by the fluid pressure at the inlet of the shunt component to radially open, thereby causing the flow regulation mechanism to be in the second state.

Preferably, the first vertical rod 213 and the second vertical rod 214 gradually converge from the annular frame toward the horizontal rod 215. With this design, the adjustment flexible tube 220 can change stepwise between the first state and the second state in response to a pressure change, thereby regulating the flow rate of the fluid passing through the shunt channel in a more rational manner. This achieves an objective of low-flow shunting under a small pressure and high-flow shunting under a large pressure, thereby enhancing safety and efficacy of atrial shunting. Of course, in practical use, a desired flow control mode can also be achieved by adjusting a material and related parameters of the adjustment flexible tube 220.

When the shunt device is implanted into an aperture in the interatrial septum, the inlet 113 of the shunt component 100 is located in the left atrium, while the flow regulation mechanism 200 and the outlet 114 are located in the right atrium. When the left atrial pressure is greater than the right atrial pressure, the blood flow enters the shunt component 100 under a pressure difference and pushes the first portion 221 radially outward to enter the right atrium, thereby achieving a shunting function. When the left atrial pressure is less than the right atrial pressure, the blood flow from the right atrium tends to flow toward the left atrium, thus collapsing the first portion 221 inward from the outside to form two sub-channels 230, which restricts further blood flow into the left atrium. Moreover, owing to the presence of the sub-channels 230, the first portions 221 are less likely to adhere to each other., thereby avoiding neointimal hyperplasia from forming from an edge of the fixing portion along an edge of the adjustment flexible tube 220, thus preventing permanent blockage of the shunt channel.

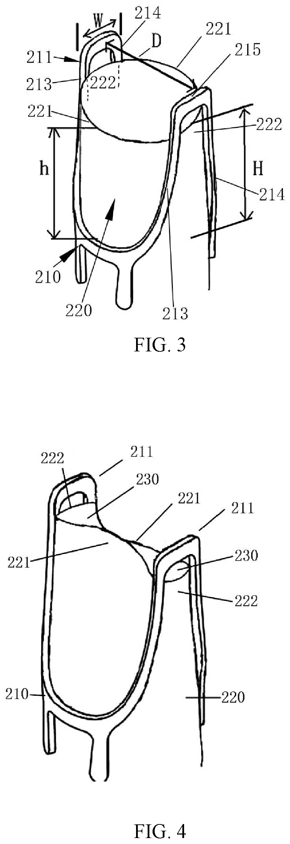

In some alternative implementations, a degree of recession of the first portion 221 can be varied by adjusting a dimension of the first portion 221, thereby modulating a degree of fluid restriction in the first state and a closure state of the first portion 221. With reference to FIG. 3 and FIG. 4, FIG. 3 is a schematic diagram of a flow regulation mechanism in a second state according to an optional implementation, and FIG. 4 is a schematic diagram of the flow regulation mechanism of FIG. 3 in a first state. Increasing the spacing between the first vertical rod 213 and the second vertical rod 214 can allow the width W of the second portion 222 to be larger, thereby enlarging the flow cross-sectional area of the sub-channel 230 in the first state. Furthermore, the extent to which the first portion 221 can ultimately be recessed is also reduced, and in an extreme case, the two first portions 221 no longer contact each other, forming a slit between them. Further, by controlling a dimension of the adjustment flexible tube 220, an optimal closure state shown in FIG. 2 can be achieved. Specifically, allowing the first portion 221 of the adjustment flexible tube 200 to have a smaller axial length dimension than an axial length dimension of the second portion 222 facilitates formation of the sub-channels 230. An edge contour of the first portion 221 is a curve and has a minimum length dimension h at an intermediate position. An edge contour of the second portion 222 is a curve connected to the edge contour of the first portion 221 and has a maximum length dimension H at an intermediate position. A ratio of h to H can be selected within a range from 0.3 to 1, depending on the requirements. Moreover, the edge contour of the first portion 221 is the curve recessed toward the inlet 113, and the edge contour of the second portion 222 is the curve that smoothly transitions into and connects to the edge contour of the first portion 221. Additionally, a part of the first portion 221 that is distal from the fixing portion 211 has a smaller axial dimension than that of a part of the first portion 221 that is proximate to the fixing portion 211. In this case, the ratio of h to H is less than 1, and the maximum length dimension H is greater than the spacing D between the two fixing portions (or the diameter of the adjustment flexible tube 220), thereby improving coaptation morphology of the covering film and reducing wrinkles. A ratio of H to D may be selected within a range from 0.5 to 3.

In some other alternative embodiments, the number of fixing portions 211 may be three or more. Correspondingly, the adjustment flexible tube 220 may be divided into three or more first portions 221 and three or more second portions, thereby forming three or more sub-channels 230. The first portions 221, acting as the blocking portion, restrict the flow rate of the fluid through the shunt channel. In some other alternative embodiments, the adjustment flexible tube 220 may also be sleeved over an exterior of the shaping frame 210. In some other alternative embodiments, the adjustment flexible tube 220 and the shunt flexible tube 120 are separate components, which may be formed by laying and/or winding two different covering films respectively.

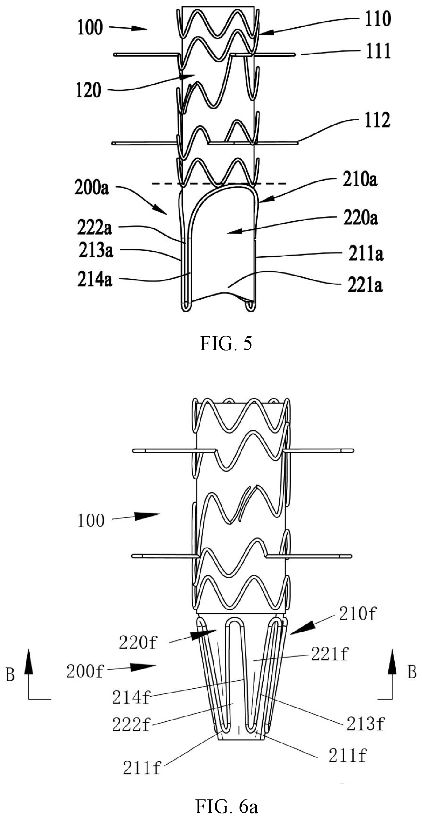

With reference to FIG. 5, FIG. 5 is a schematic structural diagram of a shunt device according to another embodiment of the present application. In the shunt device of this embodiment, the shunt component 100 is the same as the aforementioned shunt component. The flow regulation mechanism 200a includes a shaping frame 210a and an adjustment flexible tube 220a. Descriptions of other identical features are omitted here for brevity. Differences lie in that the shaping frame 210a of the flow regulation mechanism 200a is formed by bending a metal wire, rather than by cutting a metal tube. The shaping frame 210a has a frame structure having two fixing portions 211a, formed by bending a metal wire. Compared to a shaping frame formed by cutting a metal tube, the shaping frame formed by bending a metal wire offers better crimping performance and is easier to assemble and deliver. The fixing portion 211a includes a first vertical rod 213a and a second vertical rod 214a, which are formed by bending a single metal wire into an acute angle. A spacing between the two fixing portions 211a is equal to a diameter of the holder 110 in the shunt component 100. Preferably, the adjustment flexible tube 220a and the shunt flexible tube 120 in the shunt component 100 are made from a same covering film, thereby reducing additional implanted material, thus mitigating inflammatory response and lowering a risk of stenosis. The adjustment flexible tube 220a is divided by the two fixing portions 211a into a pair of first portions 221a and a pair of second portions 222a. The first portion 221a has a relatively large width (circumferential dimension) and can be recessed radially or bowed radially. The second portion 222a has a relatively small width, and the second portion 222a is fixed to the first vertical rod 213a and the second vertical rod 214a, and thus is unable to deform radially, thus maintaining a bowed state. In some alternative implementations, the adjustment flexible tube 220a may also be wrapped around an exterior of the shaping frame 210a. Even if a width of the fixing portion 211a (i.e., a spacing between the first vertical rod 213a and the second vertical rod 214a) is relatively small, or even if the first vertical rod 213a and the second vertical rod 214a are directly connected at an end without a distinct horizontal rod, the second portion 222a with a predetermined width can still be formed, and parts of the two first portions 221 proximate to the fixing portion 211 do not intersect. Preferably, similar to the previous embodiments, a part of the first portion 221a that is distal from the fixing portion 211a has a smaller axial dimension than that of a part of the first portion 221a proximate to the fixing portion 211a, so that when two opposing second portions 222a converge toward each other, a coaptation state shown in FIG. 2 may be achieved, reducing wrinkles and thereby further preventing neointimal hyperplasia.

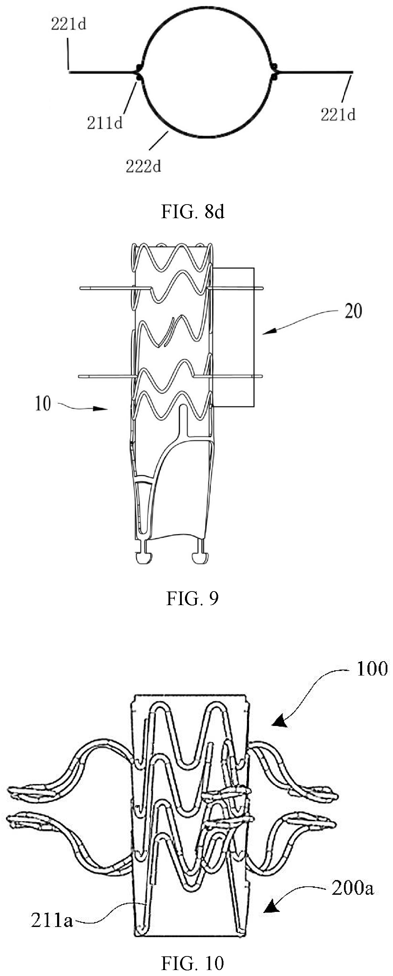

The inventors have found through research that, as shown in FIG. 1 and FIG. 10, in a case where the shaping frame 210 or 210a has a structure including two fixing portions 211 or 211a, when mobility of one first portion 221 or 221a becomes partially restricted (due to factors such as tissue hyperplasia and endothelialization), it can significantly affect flow regulation performance and may even severely compromise therapeutic efficacy of the shunt device. Based on this, the applicant, after repeated experimental verification, has designed the shunt device described in the following embodiments.

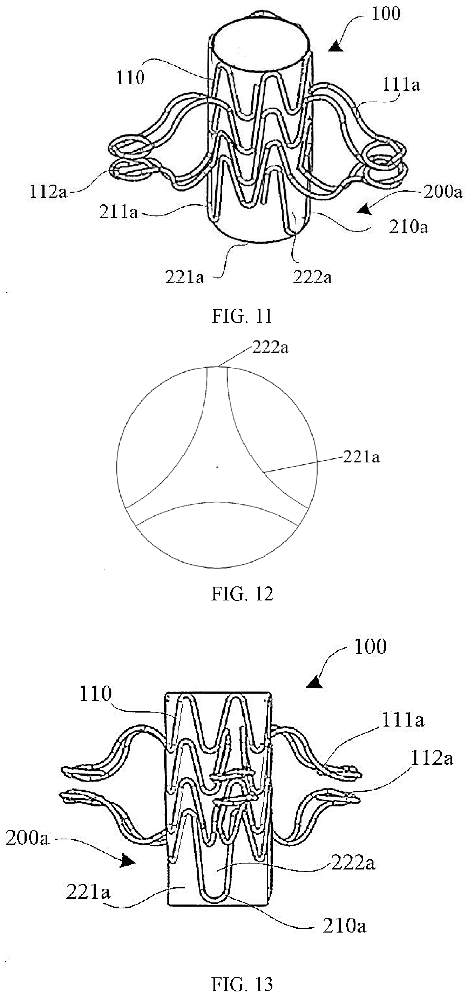

With reference to FIG. 11 to FIG. 13, FIG. 11 is a schematic structural diagram of a shunt device according to another embodiment of the present application. The shunt component 100 of the shunt device in this embodiment is similar to that in the aforementioned embodiments. Identical features will not be reiterated here. Differences lie in that the shaping frame 210a of the flow regulation mechanism 200a is a frame structure with three fixing portions 211a, formed by bending a metal wire. In some embodiments, as further illustrated in FIG. 11 to FIG. 13, the three fixing portions 211a are uniformly distributed in a circumferential direction of the adjustment flexible tube 220a, which is beneficial for applying a force uniformly to the first portions 221a, thereby contributing to an extended service life of the shunt device.

With reference to FIG. 12, compared to the structure where the shaping frame 210a has two fixing portions 211a, the adjustment flexible tube 220a is divided by the three fixing portions 211a into three first portions 221a and three second portions 222a. A force borne by each first portion 221a is relatively reduced, which is beneficial for extending a working life of the adjustment flexible tube 220a. Assuming that during cardiac contraction, an impact force from a blood flow is F, and this force is distributed uniformly on each first portion 221a, when the shaping frame 210a has two fixing portions 211a, a tension on each first portion 221a is 1/2 *F, whereas when the shaping frame 210a has three fixing portions 211a, a tension on each first portion 221a is 1/3 *F. That is to say, the greater the number of first portions 221a, the smaller the tension experienced by each first portion 221a. Therefore, its working life can be prolonged.

In addition, with reference to FIG. 2 and FIG. 12, compared to the configuration where the shaping frame 210 or 210a has two fixing portions 211 or 211a, when the shaping frame 210a has three fixing portions 211a, and mobility of any one of the first portions 221a becomes restricted (due to factors such as tissue hyperplasia and endothelialization), there are still two other first portions 221a that can function. Consequently, the adjustment flexible tube 220a can still provide a larger flow cross-sectional area in the second state, thereby ensuring a sufficient flow rate for forward shunting (blood shunting from the left atrium to the right atrium) and effectively regulating the flow rate; and the adjustment flexible tube 220a can still provide a relatively small flow cross-sectional area in the first state, thereby ensuring effective reduction of reverse shunting (blood shunting from the right atrium to the left atrium). For example, after a single first portion 221a fails (loses mobility and remains in a collapsed state), a maximum forward shunt flow rate is approximately 1/2 of an original flow rate when the shaping frame 210a has two fixing portions 211a, and a maximum forward shunt flow rate is approximately 2/3 of the original flow rate when the shaping frame 210a has three fixing portions 211a. Here, the original flow rate refers to a maximum shunt flow rate when all first portions 221a are functional and the flow regulation mechanism is in the second state. Therefore, three fixing portions provide superior capability in maintaining the forward shunting and reducing the reverse shunting compared to two fixing portions. As for the atrial shunt device, due to its relatively small overall size, configuring the adjustment flexible tube 220a to include three first portions 221a can not only extend the service life of the shunt device and maintain an effective function of the shunt device but also avoid excessive complexity in a manufacturing process.

With reference to FIG. 11, FIG. 12, and FIG. 13, the shaping frame 210a of the flow regulation mechanism 200a in FIG. 13 has three fixing portions 211a. In some embodiments, the holder 110 of the shunt component 100 and the shaping frame 210a constitute a metal frame formed by unitary winding of a wire. This integrally formed metal frame features a plurality of continuous "W"-shaped wave structures and extends in an overall helical pattern. Utilizing these "W"-shaped wave structures allows for more flexible arrangement of the shaping frame 210a of the adjustment flexible tube at the outlet of the flow regulation mechanism, thereby adjusting a closure configuration of the blocking portion and, consequently, modulating efficacy of the blocking portion on reducing reverse shunting. For example, the fixing portion of the shaping frame 210a is formed by a segment of the "W"-shaped wave structure. Compared to a frame formed by cutting, a metal frame formed by winding the wire possesses a smooth edge, which, when combined with the covering film, can effectively prevent sharp structures from damaging the adjustment flexible tube, thereby contributing to an extended service life of the shunt device.

The metal frame formed by unitary winding of the wire possesses characteristics such as easy foldability and favorable crimping, thereby enabling an overall structure to be crimped into a configuration that are unattainable when the shunt component and the shaping frame are separate, for example, a C-shape. Furthermore, an overall diameter after crimping is smaller, thereby facilitating minimally invasive surgical procedures. Moreover, after the covering film is applied to form the flexible tubes (the shunt flexible tube and the adjustment flexible tube) on the metal frame formed by unitary winding of the wire, a lumen of these tubes is smoother. This avoids a drawback of a step at a junction between the shunt component and the shaping frame that arises when the shunt component and the shaping frame are separate, thereby enhancing long-term patency of the flexible tubes, and consequently increasing the service life of the shunt device.

In scenarios where a detection module, such as a pressure measurement module, is also incorporated, the use of the metal frame formed by unitary winding of the wire can effectively minimize or prevent formation of a closed-loop structure in any cross-section, and reduces or avoids interference with and obstruction of a signal transmission of the pressure measurement module.

Preferably, an elastic wire used to make the metal frame is a nickel titanium wire, which has favorable biocompatibility and has suitable strength and flexibility.

As shown in FIG. 11 and FIG. 13, in some embodiments, the holder 110 of the shunt component 100 includes a clamping device. The clamping device includes a plurality of pairs of a first clamping arm 111a and a second clamping arm 112a, and a shunt portion. The first clamping arm 111a and the second clamping arm 112a respectively extend from the shunt portion of the holder 110 and gradually converge toward each other. The shunt portion is in close apposition with the shunt flexible tube. Furthermore, the first clamping arms 111a and the second clamping arms 112a are also integrally wound and formed with the shunt portion of the holder 110. Consequently, the first clamping arms 111a and the second clamping arms 112a can swing relative to the shunt portion along an axial direction of the shunt component 100, thereby making a junction less prone to fracture. This not only enhances a clamping force of the clamping device but also allows a spacing between the first clamping arm 111a and the second clamping arm 112a to self-adjust according to tissues of varying thickness. Preferably, the first clamping arms 111a and/or the second clamping arms 112a each includes two extension wire segments and one annular wire segment, and two ends of the annular wire segment are respectively connected to one extension wire segment, further increasing the clamping force of the clamping device.

In some other alternative embodiments, the shaping frame may also be integrally formed with the adjustment flexible tube. For example, a reinforcing structure equivalent to the fixing portion 211a is incorporated into a tube wall of the adjustment flexible tube. Such reinforcing structure may also be formed from a portion of the tube wall of the adjustment flexible tube (e.g., via thermoplastic techniques), as long as the relevant structural parts can ultimately be formed.

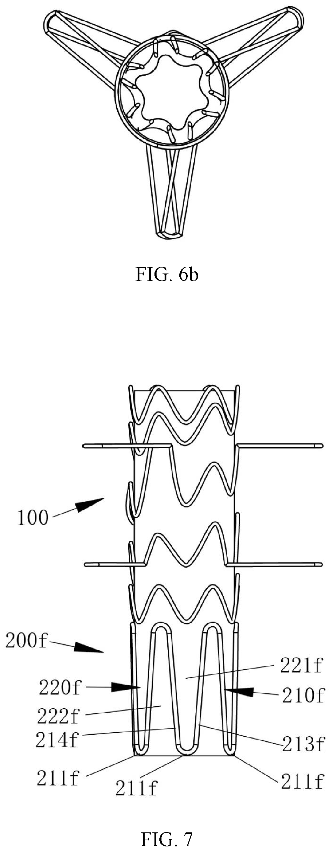

With reference to FIG. 6a and FIG. 6b, FIG. 6a is a schematic structural diagram of a shunt device according to another embodiment of the present application, and FIG. 6b is a cross-sectional view taken along the line B-B in FIG. 6a. The flow regulation mechanism is in the first state. In the shunt device of this embodiment, the shunt component 100 is the same as the aforementioned shunt component. Differences lie in that the shaping frame 210f of the flow regulation mechanism 200f is formed from an elastic wire, and the shaping frame and the adjustment flexible tube 220f are pre-formed into an initial state, which is the first state, where the adjustment flexible tube 220f has a relatively small first flow cross-sectional area. FIG. 7 is a schematic structural diagram of the flow regulation mechanism of the shunt device shown in FIG. 6a in a second state. In this case, the left atrial pressure is greater than the right atrial pressure, and under this pressure difference, a blood flow may enter the shunt component 100 and push the adjustment flexible tube 220f radially outward to enter the right atrium, thereby achieving a shunting function. When the left atrial pressure is less than the right atrial pressure, the blood flow from the right atrium tends to flow into the left atrium. Consequently, a pressure is applied to the adjustment flexible tube 220f from the outside. Simultaneously, under the effect of the pre-forming, the adjustment flexible tube 220f returns to its pre-formed state, thus restricting further blood flow into the left atrium.

The shaping frame in this embodiment includes a plurality of elastic wires. These elastic wires are interconnected and are pre-formed into an initial state via a pre-forming process. In the initial state, ends of the elastic wires are interconnected, and an orthographic projection of these ends may be linked to form a circle with a predetermined diameter. This predetermined diameter matches a diameter of the adjustment flexible tube. Other ends of the elastic wires are also interconnected, thereby forming at least two elastic guide frames 211f. The elastic guide frame 211f extends in an axial direction and gradually approaches a central axis, thereby forming an elastic cantilever that are inclined toward the central axis. Preferably, these elastic wires are integrally formed, i.e., formed from a single wire. The shaping frame is fixed to the adjustment flexible tube through processes such as stitching or heat pressing. Constrained by the elastic guide frames 211f, the first portion 221f of the adjustment flexible tube is configured to be capable of being recessed radially or bowed radially, thereby serving as the blocking portion. In contrast, the second portion 222f is not constrained by the elastic guide frames 211f and maintains in a bowed state.

In the initial state, the flow regulation mechanism is in the first state, i.e., a restricted flow state. In this embodiment, the shaping frame 210f comprises six elastic guide frames 211f, thereby dividing the adjustment flexible tube 220f into twelve segments: six first portions 221f and six second portions 222f. In the first state, a petal-shaped structure is formed. The elastic guide frame 211f includes a first vertical rod 213f and a second vertical rod 214f. The first portion 221f is located between the first vertical rod 213f and the second vertical rod 214f. The second portion 222f is located between two adjacent elastic guide frames 211f. As shown in FIG. 7, when blood flows from the shunt channel through an inlet of the shunt component 100 out of the flow regulation mechanism 200f, the blood can push the shaping frame to expand radially outward, and the adjustment flexible tube is gradually bowed in response to a change in a pressure difference between two sides until the adjustment flexible tube returns to a tubular shape, so that the shunt channel attains a second flow cross-sectional area, and the flow regulation mechanism 200f is in the second state, i.e., a permitted flow state.

It is to be understood that in other embodiments, some of the second portions 222f of the adjustment flexible tube 220f may also be omitted. In other words, the adjustment flexible tube may also include only the first portion 221f that forms the blocking portion. A transition of the flow regulation mechanism 200f between the first and second states is achieved through radial swinging of the first portion 221f. When a fluid pressure inside the shunt component 100 is less than a fluid pressure external to the flow regulation mechanism 200f, the first portion 221f is driven by the pressurized fluid external to the flow regulation mechanism 200f to collapse radially, thereby placing the flow regulation mechanism 200f in the first state. When the fluid pressure inside the shunt component 100 is greater than the fluid pressure external to the flow regulation mechanism 200f, the first portion 221f is driven by the pressurized fluid inside the shunt component 100 to open radially, thereby causing the flow regulation mechanism 200f to be in the second state. Specifically, when the first portion 221f is in an initial state, i.e., a collapsed state, the flow regulation mechanism 200f is in the first state. When the first portion 221f is subjected to a radially outward force to move away from a central axis, the first portion 221f is in an open state, and the flow regulation mechanism 200f is in the second state. In this case, a first flow cross-sectional area of the shunt channel can be adjusted by modifying adjacent elastic guide frames.

In other embodiments, an elastic sheet may be used in place of the elastic wire. The elastic guide frame may also be sheet-shaped or consist of individual elastic wires, fixed to an annular frame. The blocking portion may also be fabricated from other materials and is not limited to a soft, deformable material, as long as the blocking portion can achieve axial extension gradually approaching the central axis in the initial state and move away from the central axis when subjected to a radially outward force.

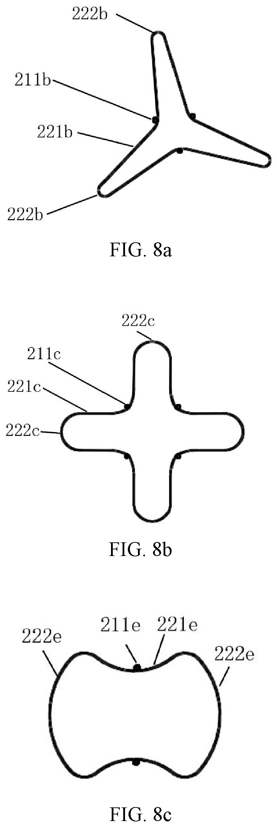

With reference to FIG. 8a to FIG. 8d, schematic diagrams of the flow regulation mechanism in various optional configurations are illustrated therein.

FIG. 8a is a schematic structural diagram of a flow regulation mechanism according to an optional implementation. In this implementation, the shaping frame includes three elastic guide frames 211b uniformly distributed in a circumferential direction. A proximal end of the adjustment flexible tube is circular and is connected to the shunt component, while a distal end of the adjustment flexible tube is shaped into a Y-shaped configuration by being pressed toward the central axis by the three elastic guide frames 211b. In other words, an initial state of the flow regulation mechanism is the first state, i.e., a restricted flow state. Three second portions 222b at the distal end of the adjustment flexible tube remain in their original shape. The first portion 221b located between two adjacent second portions 222b is compressed by the elastic guide frame 211b. One elastic guide frame 211b is formed by two elastic wires. An end of the elastic wire is fixed to a circumferential surface of the shunt component, and another end of the elastic wire extends axially and gradually approaches a central axis to form an elastic cantilever inclined toward the central axis. The elastic guide frame 211b is capable of being parallel to the central axis when subjected to a radially outward force. The three elastic guide frames 211b cause a portion of the adjustment flexible tube to collapse toward a center of the shunt channel, thereby forming a Y-shaped outlet. When the pressurized fluid from the shunt component flows through this Y-shaped flow cross-section, the fluid pressure can drive the first portion 221b and the elastic guide frame 211b to expand outward, thereby increasing a flow cross-sectional area. After the pressurized fluid in this direction ceases, the elastic guide frame 211b drives the first portion 221b back to its original position, thereby restoring the flow regulation mechanism to the first state and restricting a flow of a fluid in a reverse direction.

FIG. 8b is a schematic structural diagram of a flow regulation mechanism according to another optional implementation. In this embodiment, the flow regulation mechanism is similar to the structure shown in FIG. 8a, with differences being that the number of the elastic guide frames 211c is four, the number of the first portions 221c is also four, and the number of the second portions 222c is also four. In the first state, a shape of a flow cross-section formed by a distal end of the adjustment flexible tube is X-shaped.

FIG. 8c is a schematic structural diagram of a flow regulation mechanism according to another optional implementation. In this embodiment, a width of the second portion 222e is substantially equal to a width of the first portion 221e. There are two elastic guide frames 211e, and a spacing between their distal ends is relatively large. Consequently, a spacing between two first portions 221e is relatively large, resulting in a relatively weak restrictive effect on the pressurized fluid.

FIG. 8d is a schematic structural diagram of a flow regulation mechanism according to another optional implementation. In this embodiment, there are four elastic guide frames 211d. These four elastic guide frames 211d form two pairs of clamping structures. The two pairs of clamping structures are uniformly distributed in a circumferential direction. Within the clamping structure, two elastic guide frames 211d are spaced closely together, thereby completely converging a portion of the adjustment flexible tube located between the two elastic guide frames 211d. This portion is the first portion 221d. The second portion 222d, located between the two pairs of clamping structures, forms a shunt channel with a relatively small diameter. The adjustment flexible tube can expand under the push of the pressurized fluid from the shunt tube (simultaneously pushing the elastic guide frame 211d), thereby opening the first portion 221d.

In other embodiments, a suitable material may also be selected and pre-formed to create the blocking portion (i.e., the shaping frame and the blocking portion are integrally formed), thereby enabling the blocking portion to transition between a collapsed state and an open state, as long as a first flow cross-sectional area corresponding to a collapsed configuration of the blocking portion is less than a second flow cross-sectional area corresponding to an open configuration of the blocking portion, and the first flow cross-sectional area is greater than zero (the flow regulation mechanism is not completely closed).

It is to be understood that in the aforementioned embodiments, at any given moment, when the fluid pressure at the inlet of the shunt component and/or inside the shunt component is equal to the fluid pressure external to the flow regulation mechanism, the blocking portion may remain in a state determined by a pressure gradient present at the previous moment. For instance, at the previous moment, when the fluid pressure inside the shunt component is greater than the fluid pressure external to the flow regulation mechanism, the blocking portion is driven by the fluid pressure inside the shunt component to open radially, thereby placing the flow regulation mechanism in the second state, and then at the next moment, when the fluid pressure inside the shunt component is equal to the fluid pressure external to the flow regulation mechanism, the blocking portion maintains in the second state.

With reference to FIG. 9, FIG. 9 is a schematic structural diagram of a shunt device according to another embodiment of the present application. In this embodiment, the shunt device includes a shunt 10 and a sensor 20. The shunt 10 is the shunt device described in the preceding embodiments. The monitoring device is configured to monitor human physiological parameters such as a pressure, a blood viscosity, and a flow rate. The monitoring device may be, for example, a wireless pressure sensor capable of monitoring a pressure within the heart or blood vessels. The monitoring device is fixed side-by-side and tangentially to an outer periphery of the shunt component of the shunt 10. The shunt 10 and the sensor 20 can be connected together by means such as crimping with a sleeve, bonding, stitching, or welding, thereby enabling simultaneous therapeutic and diagnostic functions after implantation into the human body.

The foregoing descriptions are merely specific implementations of the present application. However, the protection scope of the present application is not limited thereto. Any person skilled in the art may readily conceive of modifications or substitutions within the technical scope disclosed in the present application, and such modifications or substitutions shall fall within the protection scope of the present application. Consequently, the protection scope of the present application shall be defined by the appended claims.

## Claims

1. A shunt device for shunting a pressurized fluid, comprising:
a shunt component comprising a body, and an inlet and an outlet located at both ends of the body, the shunt component defining a shunt channel for the pressurized fluid; and
a flow regulation mechanism connected to the shunt component and configured to regulate a flow rate of the pressurized fluid flowing through the shunt channel, wherein the flow regulation mechanism comprises a deformable blocking portion configured to be driven by a fluid pressure external to the flow regulation mechanism to collapse radially when a fluid pressure at the inlet of the shunt component is less than the fluid pressure external to the flow regulation mechanism.

2. The shunting device according to claim 1, wherein the blocking portion is configured to, when a fluid pressure inside the shunt component is less than the fluid pressure external to the flow regulating mechanism, be driven by the fluid pressure external to the flow regulating mechanism to collapse radially, thus allowing the flow regulation mechanism to never completely close the shunt channel.

3. The shunt device according to claim 1 or 2, wherein
the blocking portion is configured to vary a flow cross-sectional area of the shunt channel, and the flow regulation mechanism has a first state and a second state; in the first state, the blocking portion is in a collapsed configuration such that the shunt channel has a first flow cross-sectional area, and in the second state, the blocking portion is in an open configuration such that the shunt channel has a second flow cross-sectional area; and the first flow cross-sectional area is greater than zero and is less than the second flow cross-sectional area; and
the blocking portion is configured to, when a fluid pressure at the inlet of the shunt component and/or inside the shunt component is less than the fluid pressure external to the flow regulation mechanism, be driven by the fluid pressure external to the flow regulation mechanism to collapse radially, thereby causing the flow regulation mechanism to be in a first state.

4. The shunt device according to claim 3, wherein when the blocking portion is in a collapsed configuration, the blocking portion is deformed into a recessed state, and when the blocking portion is in an open configuration, the blocking portion is deformed into a bowed state; and the blocking portion is configured to, when the fluid pressure at the inlet of the shunt component and/or inside the shunt component is less than the fluid pressure external to the flow regulation mechanism, be driven by the fluid pressure external to the flow regulation mechanism to be recessed radially, thereby causing the flow regulation mechanism to be in the first state.

5. The shunt device according to any one of claims 1 to 4, wherein the flow regulation mechanism comprises an adjustment flexible tube connected to the shunt component, a tube wall of the adjustment flexible tube comprises a first portion distributed in a circumferential direction, the first portion is configured to be recessed radially or bowed radially, and the first portion is the blocking portion.

6. The shunt device according to claim 5, wherein the adjustment flexible tube further comprises a second portion, and the second portion and the first portion are distributed spaced apart from each other in the circumferential direction; and the flow regulation mechanism further comprises a shaping frame, and the shaping frame is configured to maintain the second portion in a bowed state and to allow the first portion to be controllably recessed or bowed with a constraint of the shaping frame by supporting a predetermined portion of the adjustment flexible tube.

7. The shunt device according to claim 6, wherein the shaping frame comprises a plurality of fixing portions, an end of the fixing portion is connected to the shunt component, another end of the fixing portion extends in an axial direction, and the plurality of fixing portions are distributed in a circumferential direction of the adjustment flexible tube; and a tube wall of the adjustment flexible tube comprises a plurality of first portions and a plurality of second portions corresponding to the plurality of fixing portions, the first portion is disposed between two circumferentially adjacent fixing portions, and the second portion is fixed to the fixing portion and has a predetermined width, such that parts of two adjacent first portions that are proximate to the fixing portion remain out of contact with each other.

8. The shunt device according to claim 7, wherein a width of the fixing portion corresponds to a width of the second portion, and the adjustment flexible tube is disposed at an inner side and/or an outer side of the fixing portion.

9. The shunt device according to claim 7 or 8, wherein the fixing portion comprises a first vertical rod and a second vertical rod connected to each other, a predetermined spacing is provided between the first vertical rod and the second vertical rod, an end of the first vertical rod and an end of the second vertical rod are connected to the shunt component, and another end of the first vertical rod and another end of the second vertical rod gradually converge along extension directions of the first vertical rod and the second vertical rod.

10. The shunt device according to claim 9, wherein the fixing portion further comprises a horizontal rod connecting the another end of the first vertical rod and the another end of the second vertical rod, a reinforcement rib disposed between the first vertical rod and the second vertical rod, and an annular frame connecting the end of the first vertical rod and the end of the second vertical rod.

11. The shunt device according to any one of claims 7 to 10, wherein the plurality of fixing portions comprises three fixing portions.

12. The shunt device according to claim 11, wherein the three fixing portions are uniformly distributed in the circumferential direction of the adjustment flexible tube.

13. The shunt device according to any one of claims 7 to 12, wherein a hanging lug is provided at distal end of the fixing portion, and the hanging lug is configured to connect to a predetermined structure within an interventional catheter.

14. The shunt device according to any one of claims 7 to 13, wherein an edge contour of the first portion is a curve recessed toward the inlet, an edge contour of the second portion is a curve smoothly connected to the edge contour of the first portion, and a part of the first portion that is distal from the fixing portion has an axial dimension that is less than an axial dimension of a part of the first portion that is proximate to the fixing portion.

15. The shunt device according to any one of claims 6 to 14, wherein an edge contour of the first portion is a curve and has a minimum length dimension at an intermediate position, an edge contour of the second portion is a curve connected to the edge contour of the first portion and has a maximum length dimension at an intermediate position, and a ratio of the minimum length dimension to the maximum length dimension ranges from 0.3 to 1.

16. The shunt device according to claim 15, wherein a ratio of the minimum length dimension to a diameter of the adjustment flexible tube ranges from 0.5 to 3.

17. The shunt device according to any one of claims 6 to 16, wherein there are a plurality of first portions, the plurality of first portions, when recessed, maintain incomplete contact at a center of the shunt channel, thereby dividing the shunt channel into a plurality of sub-channels, and the plurality of sub-channels are located at an edge of the shunt channel.

18. The shunt device according to any one of claims 6 to 16, wherein there are a plurality of first portions, and the plurality of first portions, when recessed, maintain out of contact with each other and form a slit.

19. The shunt device according to any one of claims 6 to 18, wherein the shunt component and the shaping frame are formed as a metal framework by unitary winding of a wire, and any cross-section of the metal frame has a non-enclosed contour.

20. The shunt device according to any one of claims 6 to 18, wherein the shaping frame is integrally formed with the blocking portion; or the shaping frame is connected to the blocking portion as a separate component, and the separate component is formed by cutting a metal tube or by bending a metal wire.

21. The shunt device according to any one of claims 5 to 20, wherein the body comprises a holder and a shunt flexible tube disposed at an inner surface and/or an outer surface of the holder, the shunt flexible tube and the adjustment flexible tube are integrally formed, the shunt component is connected to the flow regulation mechanism at the outlet, the body and the shunt flexible tube form a first segment of the shunt channel, and the adjustment flexible tube forms a second segment of the shunt channel.

22. The shunt device according to claim 21, wherein the holder comprises a clamping device and a shunt portion, the clamping device comprises a plurality of pairs of a first clamping arm and a second clamping arm, and the first clamping arm and the second clamping arm respectively extend from the shunt portion.

23. The shunt device according to claim 22, wherein the first clamping arm and the second clamping arm respectively extend from the shunt portion of the holder and gradually converge toward each other.

24. The shunt device according to claim 23, wherein first clamping arms and/or second clamping arms each comprises two extension wire segments and an annular wire segment, and two ends of the annular wire segment are respectively connected to one extension wire segment.

25. The shunt device according to claim 4, wherein the flow regulation mechanism further comprises a shaping frame, the shaping frame comprises a plurality of elastic guide frames, and the elastic guide frames are connected to the blocking portion and are configured to maintain the blocking portion in the collapsed configuration in an initial state.

26. The shunt device according to claim 25, wherein an end of the elastic guide frame is connected to the body, another end of the elastic guide frame forms an elastic cantilever in an axial direction, and the another end is closer to a center of the shunt channel than the end.

27. The shunt device according to claim 26, wherein the flow regulation mechanism comprises an adjustment flexible tube connected to the shunt component, a tube wall of the adjustment flexible tube comprises a first portion and a second portion distributed in a circumferential direction, and the second portion and the first portion are distributed spaced apart from each other in the circumferential direction; and the first portion is configured to be capable of being recessed radially or bowed radially, the first portion is the blocking portion, and the elastic guide frames are configured to maintain the second portion in a constantly bowed state.

28. The shunt device according to any one of claims 25 to 27, wherein the elastic guide frame is formed by bending a metal wire and, when subjected to a radially outward force, is capable of being parallel to a central axis of the shunt component.

29. The shunt device according to any one of claims 4 to 28, wherein there are a plurality of blocking portions, and an initial state of the flow regulation mechanism is the first state or the second state.

30. The shunt device according to any one of claims 1 to 29, wherein the shunt device further comprises a monitoring device, and the monitoring device is connected side by side with the shunt component.
